Europäisches Patentamt

European Patent Office

Office européen des brevets

(11) Numéro de publication: **0 266 258 B1**

## FASCICULE DE BREVET EUROPEEN

(45) Date de publication de fascicule du brevet: **28.04.93** (51) Int. Cl.5: **C12P 7/56**, C12N 1/20

(21) Numéro de dépôt: **87402307.0**

(22) Date de dépôt: **16.10.87**

(54) **Procédé de préparation d'acide lactique.**

(30) Priorité: **29.10.86 FR 8615254**

(43) Date de publication de la demande:
**04.05.88 Bulletin 88/18**

(45) Mention de la délivrance du brevet:
**28.04.93 Bulletin 93/17**

(84) Etats contractants désignés:
**AT BE CH DE ES FR GB GR IT LI LU NL SE**

(56) Documents cités:
**EP-A- 0 190 770
FR-A- 490 589
FR-A- 2 209 838
US-A- 2 474 046**

**CHEMICAL ABSTRACTS, vol. 103, no. 17, 28 octobre 1985, page 399, abrégé no. 138296c, Columbus, Ohio, US; E.B. LEE et al.: "Effect of the fish meat hydrolyzate on the growth of lactic acid bacteria", & NONGOP KISUL YONGU POGO (CHUNGNAM TAEHAKKYO) 1984, 11(1), 120-32**

**CHEMICAL ABSTRACTS, vol. 78, no. 1, 8 janvier 1973, page 243, abrégé no. 2765n, Columbus, Ohio, US; & JP-A-72 38 192 (M. TAKEUCHI et al.) 27-09-1972**

**CHEMICAL ABSTRACTS, vol. 101, no. 21, 19 novembre 1984, page 607, abrégé no. 189945e, Columbus, Ohio, US; H. YUGUCHI: "Use of fish meat hydrolyzate for fermented milk products. VII. Correlations between the chemical composition of fish meat hydrolyzate and stimulatory activity for the growth of lactic acid bacteria and decrease of curd tension of fermented milk products" & RAKUNO KAGAKU, SHOKUHIN NO KENKYU 1984, 33(3), A81-A91**

(73) Titulaire: **RHONE-POULENC CHIMIE
25, quai Paul Doumer
F-92408 Courbevoie Cédex(FR)**

(72) Inventeur: **Jarry, Alain
Le Paradis Maisonnet
F-79500 Melle(FR)**
Inventeur: **Lamonerie, Hubert
rue Foucaudrie
F-79500 Melle(FR)**

(74) Mandataire: **Dutruc-Rosset, Marie-Claude et al
RHONE-POULENC CHIMIE Direction des Brevets 25, Ouai Paul Doumer
F-92408 Courbevoie Cédex (FR)**

EP 0 266 258 B1

## Description

La présente invention concerne un procédé de préparation industriel d'acide lactique. Elle concerne plus particulièrement un procédé de préparation industriel d'acide lactique optiquement actif par fermentation de sources hydrocarbonées par des microorganismes appartenant au genre Lactobacillus.

La préparation d'acide lactique par fermentation de milieux hydrocarbonés est connue depuis le début du siècle. Il est décrit par exemple dans le brevet français N° 679418 de 1929 un procédé de préparation d'acide lactique par fermentation de moûts contenant des sucres à l'aide de microorganisme appartenant au genre lactobacillus.

Depuis cette époque, de nombreuses améliorations aux procédés de préparation d'acide lactique par exemple par addition de peptones, d'extraits de levure (EP 113215), de levures de bière (EP 69291) ont permis d'augmenter la productivités qui restent néanmoins presque toujours inférieures à 100 g/l.

Par ailleurs, il est connu selon Chemical Abstracts 103, 138296c (1985), 78, 2765n (1973) et 101, 189945e (1984) d'utiliser un hydrolysat de poisson (maquereaux, sardines), dans la fermentation de l'acide lactique.

On a décrit dans FR-A 490586, l'utilisation d'un jus brut extrait de la betterave à sucre et de la mélasse dans la préparation de l'acide lactique.

Aucun des procédés décrits dans l'art antérieur ne permet une productivité satisfaisante. On entend par productivité le poids d'acide lactique formé par heure et par litre de milieu nutritif.

La présente invention permet d'obtenir des productivités atteignant et même dépassant 110 g par litre. Comme mentionné précédemment, aucun des procédés de l'art antérieur ne permettait jusqu'alors d'atteindre 110 g/l/h même en augmentant la teneur en sucre de la solution initiale.

Dans EP-A 0190770, on a mentionné l'obtention de l'acide lactique D optiquement pur, par fermentation à l'aide de lactobacillus d'un milieu nutritif contenant une source hydrocarbonée, un sel organique, un promoteur de croissance, tel que les extraits de levure, la peptone, l'extrait de viande et la poudre de soja, et un agent neutralisant.

Dans ce cas, l'acide lactique D est obtenu avec un rendement pouvant atteindre jusqu'à 128 g/l.

La présente invention a permis d'atteindre des solutions beaucoup plus concentrées en acide lactique tout en conservant des temps de fermentation corrects de l'ordre de 60 heures.

L'invention a donc pour objet un procédé de préparation d'acide lactique D ou L optiquement pur par fermentation d'un milieu nutritif qui contient une source hydrocarbonée, une source azotée, de l'acide phosphorique, des vitamines, un agent neutralisant à l'aide d'un microorganisme appartenant à la classe des lactobacillus caractérisé en ce que la source hydrocarbonée est composée de sirop "O" et en ce qu'on ajoute au milieu nutritif, une source complémentaire d'azote qui est un protéolysat de poisson.

La source hydrocarbonée utilisée dans le procédé de la présente invention pour être d'origine diverse. Elle peut être notamment constituée de saccharose, de lactose, de mélasse, de glucose, d'amidon, de maltose.

La source azotée elle aussi peut être d'origine très diverse. On peut citer non limitativement l'eau de lavage de maïs, concentrée, ("corn steep liquor"), la levure de bière telle que notamment ses hydrolysats comme le "Yeast extract" ou la levure fraîche, les peptones ou le chlorure d'ammonium. On préfère mettre en oeuvre l'eau de lavage de maïs, concentrée ("corn steep liquor").

L'agent neutralisant qui permet de maintenir le PH de la solution nutritive aux environs de 6 pendant toute la période de croissance et d'activité synthétique du microorganisme est choisi parmi les hydroxydes alcalins, alcalinoterreux ou d'ammonium ainsi que parmi les carbonates. On préfère pour une meilleure mise en oeuvre de l'invention utiliser comme agent neutralisant pendant la phase de croissance du microorganisme la soude et pendant sa phase d'activité l'ammoniaque ou l'ammoniac.

Le microorganisme utilisé pour la préparation d'acide lactique D ou L optiquement pur est choisi parmi l'ensemble des microorganismes appartenant à la classe des lactobacillus.

On peut notamment citer pour la préparation d'acide lactique D (-) lévogyre : Lactobacillus lactis ATCC 12314, Lactobacillus delbrueckii NCDO (National Collection of Dairy Organism) 1744, Lactobacillus leichmanii ATCC 4797, Lactobacillus bulgaricus ATCC 11842, Lactobacillus lactis ATCC 11061, Lactobacillus jugurt ATCC 9224. Pour la préparation d'acide lactique L(+) dextrogyre on peut citer non limitativement : Lactobacillus casei subsp. rhamnosus ATCC 7469, Lactobacillus casei ATCC 11443 ; Lactobacillus delbrueckii NR LL B 445.

La source azotée complémentaire objet de la présente invention est un protéolysat de poissons.

Ce protéolysat est obtenu par hydrolyse de blancs et de maigre de poissons à l'aide de protéase. Il est vendu sous la marque SEAH-FISH-D.A par la société Seah International. Mais tout autre protéolysat de poisson dégraissé peut être utilisé dans le cadre de la présente invention. Ce protéolysat peut être utilisé

2

frais ou sous forme sèche notamment après atomisation.

Le protéolysat de poisson est utilisé selon des concentrations comprises entre 3 et 5 g par litre de milieu nutritionel et de préférence d'environ 3,5 g par litre. Des quantités supérieures ne sont pas à exclure du procédé selon l'invention, elles n'apportent cependant aucun avantage supplémentaire. L'homme de l'art adaptera la quantité optimale de protéolysat de poisson à utiliser en fonction de l'économie du procédé.

La source hydrocarbonée est composée de sirop "O". On entend par sirop "O" le sirop concentré qui sort de la dernière concentration dans les procédés d'extraction du sucre de la betterave. Ce sirop "O" contient environ 60 à 65 % de saccharose.

L'extraction de sucre à partir de la betterave consiste dans une première étape à couper les betteraves en lanières ou "cossettes".

Dans une deuxième' étape on fait diffuser le sucre contenu dans ces lanières par passage à contre courant de ces lanières d'eau maintenue à environ 70-80° C. L'eau sortant du circuit d'extraction à contre courant contient le sucre ainsi que diverses impuretés contenues dans les betteraves. Les impuretés sont éliminées par mise en contact de la solution aqueuse avec de la chaux (chaulage), la solution obtenue est mise en contact avec du gaz carbonique (carbonatation)qui permet la précipitation de carbonate de calcium et des impuretés. Après filtration on opère une deuxième carbonatation jusqu'à complète précipitation de la chaux dissoute, puis une deuxième filtration.

La solution aqueuse épurée contenant environ 13 % de saccharose est concentrée jusqu'à environ 60 à 65 % de saccharose. Cette solution constitue le sucre "O". Elle contient une forte proportion de saccharose et une partie des constituants de la mélasse. Elle présente l'avantage lorsqu'elle est utilisée comme source nutritive dans la production d'acide lactique d'apporter une meilleure productivité que l'utilisation du saccharose pur ou du mélange saccharose-mélasse. Cet avantage tout à fait surprenant associé à l'augmentation de productivité apportée par l'utilisation d'une source d'azote complémentaire permet d'obtenir des productivités de l'ordre de 130 grammes d'acide lactique par litre de milieu nutritif qui n'avaient jamais été atteintes dans l'art antérieur.

L'invention va être décrite plus complétément à l'aide des exemples suivants que ne doivent en aucun cas être considérés comme limitatifs de l'inventin. Cette dernière comprend tous les moyens constituant des équivalents techniques des moyens décrits ainsi que leurs combinaisons si celles-ci sont mises en oeuvre dans le cadre de la protection comme revendiquée.

Exemple 1 à 12 :

A partir d'une souche de Lactobacillus lactis ATCC 12314, on ensemence avec 0,5 % en poids de souche un erlenmeyer contenant un milieu nutritif composé de :

| mélasse | 100 gl |
|---|---|
| extrait de levure | 6 g/l |
| peptone pancréatique de caséine | 5 g/l |
| $CH_3COONa, 3 H_2O$ | 10 g/l |
| sulfate de magnésium, $7H_2O$ | 0,2 g/l |
| sulfate de manganèse, $H_2O$ | 0,01 g/l |
| sulfate ferreux, $7H_2O$ | 0,01 g/l |
| acid phosphorique | 0,05 ml/l |
| tween 80 | 1 ml/l |
| vitamine B 12 | 20 mg/l |
| antimousse | quelques gouttes |
| $CaCo_3$ | 36 g/l |
| eau | quantité suffisante pour 1 litre |

Après ensemencement, on laisse incuber 16 heures à l'étuve à 40° C.

A partir de cet erlenmeyer incubé on ensemence avec 0,5 % en poids de la 1ère culture une cuve de 50 litres contenant un milieu nutritif composé de :

EP 0 266 258 B1

| mélasse | 100 g/l |
| eau de lavage de maïs concentrée | 35 g/l |
| tween 80 | 1 ml/l |
| CaCO$_3$ | 36 g/l |
| eau | quantité suffisante pour 1 litre |

Après ensemencement on laisse incuber 8 heures à 40° C.

A partir de cet inoculum, on ensemence avec 3,5 % en poids d'inoculum, divers milieux de culture d'une contenance de 1000 litres dont les compositions sont indiquées dans le tableau. Le PH est maintenu à 5,8 par addition d'ammoniaque, d'ammoniac ou de carbonate.

Les exemples 1, 2, 3, 4, 5, 6, 7, sont des exemples comparatifs dans lesquels la source d'azote complémentaire est un extrait de levure (exemples 1 et 3) ou des exemples dans lesquels aucune source complémentaire n'est utilisée(exemples 2, 4, 5 et 7).

Les exemples (1 et 4) sont des exemples comparatifs dans lesquels la source hydrocarbonée est du glucose, les exemples 2 et 3 sont des exemples comparatifs dans lesquels la source hydrocarbonée est le saccharose, les exemples 5, 6, 7 et 8 sont des exemples dans lesquels la source hydrocarbonée est un mélange saccharose-mélasse (75/25).

Les exemples selon l'invention 6 et 8 prouvent que la présence de protéolysat de poisson augmente le rendement quelque soit la source hydrocarbonée et les exemples 9 à 12 prouvent que la présence simultanée de protéolysat de poisson comme source azotée complémentaire et de sirop "O" comme source hydrocarbonée apporte un effet synergique qui se traduit par une productivité nettement améliorée.

Acide lactique D-

| ESSAI No. | Source carbonée | | | | initiale sucre g/l | initiale g/l | Source azotée CSL g/l | Proteo-lysat g/l | Extrait levures g/l | Foult-Springer NH4OH | Neutralisation NH3 | CaCO3 | Acide lactique Produit g/l | Durée Fermentation heures |
|---|---|---|---|---|---|---|---|---|---|---|---|---|---|---|
| | Glucose | Saccharose | Mélasse | Sirop "O" | | | | | | | | | | |
| 1 | 100 % | | | | 110 | 130 | | | | | | | 110 | 63 |
| 2 | | 100 % | | | 100 | 150 | | | | | | | 104,4 | 87 |
| 3 | | 100 % | | | 100 | 150 | | | 3 | | | | 102 | 87 |
| 4 | 100 % | | | | 110 | 150 | | | | | | | 97 | 60 |
| 5 | | 75 % | 25 % | | 100 | 135 | | | 5 | | | + | 90,8 | 65 |
| 6 | | 75 % | 25 % | | 127,5 | 135 | | 3,5 | | | | + | 112,3 | 62 |
| 7 | | 75 % | 25 % | | 95 | 135 | | | | + | | + | 92,4 | 55 |
| 8 | | 75 % | 25 % | | 115 | 132 | | 3,2 | | + | + | + | 116 | 75 |
| 9 | | | | 100 % | 130 | 132,5 | | 3,25 | | | + | | 131,6 | 60 |
| 10 | | | | 100 % | 140 | 145 | | 3,5 | | | + | | 130,5 | 63 |
| 11 | | | | 100 % | 135 | 135 | | 5,37 | | + | + | | 133,7 | 70 |
| 12 | | | | 100 % | 135 | 140 | | 4 | | | + | | 138 | 70 |

CSL = "Corn steep liquor" = eau de lavage de maïs concentrée.

## Revendications

1. Procédé de préparation d'acide lactique D ou L optiquement pur par fermentation d'un milieu nutritif qui contient une source hydrocarbonée, une source azotée, de l'acide phosphorique, des vitamines, un agent neutralisant , à l'aide d'un microorganisme appartenant à la classe des lactobacillus caractérisé en ce que la source hydrocarbonée est composée de sirop "O" et en ce qu'on ajoute au milieu nutritif, une source complémentaire d'azote qui est un protéolysat de poisson.

2. Procédé selon la revendication 1, caractérisé en ce que le protéolysat de poisson est utilisé à une concentration comprise entre 3 et 5 g par litre de milieu nutritif.

3. Procédé selon la revendication 1, caractérisé en ce que la source azotée est composée d'eau de lavage de maïs, concentrée "corn steep liquor" et de protéolysat de poisson.

**Claims**

1. Process for the preparation of optically pure D or L lactic acid by the fermentation of a nutrient medium containing a hydrocarbon source, a nitrogen source, phosphoric acid, vitamins and a neutralising agent, using a microorganism which belongs to the Lactobacillus class, characterised in that the hydrocarbon source consists of syrup "O" and in that a supplementary nitrogen source, which source is a fish proteolysate, is added to the nutrient medium.

2. Process according to Claim 1, characterised in that the fish proteolysate is employed at a concentration of between 3 and 5 g per litre of nutrient medium.

3. Process according to Claim 1, characterised in that the nitrogen source consists of the concentrated corn-washing liquor "corn steep liquor" and fish proteolysate.

**Patentansprüche**

1. Verfahren zur Herstellung optisch reiner D- oder L-Milchsäure durch Fermentation auf einem Nährboden, der eine Kohlehydratquelle, eine Stickstoffquelle, Phosphorsäure, Vitamine und einen Neutralisator enthält, mit Hilfe eines zur Klasse der Lactobacilli gehörenden Mikrooganismus, dadurch gekennzeichnet, daß die Kohlehydratquelle aus Sirup "O" besteht und daß zum Nährboden eine zusätzliche Stickstoffquelle, nämlich ein Fischproteolysat, zugegeben wird.

2. Verfahren nach Anspruch 1, dadurch gekennzeichnet, daß das Fischproteolysat in einer Konzentration zwischen 3 und 5 g pro Liter Nährboden angewendet wird.

3. Verfahren nach Anspruch 1, dadurch gekennzeichnet, daß die Stickstoffquelle aus konzentriertem Mais-Waschwasser ("corn steep liquor") und aus Fischproteolysat besteht.